## Europäisches Patentamt

## European Patent Office

## Office européen des brevets

(11) Publication number: **0 010 812**
**B1**

(12) **EUROPEAN PATENT SPECIFICATION**

(45) Date of publication of patent specification: **07.03.84**

(51) Int. Cl.³: **A 61 F 5/47**

(21) Application number: **79200623.1**

(22) Date of filing: **26.10.79**

(54) Contraceptive device.

(30) Priority: **26.10.78 NL 7810696**

(43) Date of publication of application:
**14.05.80 Bulletin 80/10**

(45) Publication of the grant of the patent:
**07.03.84 Bulletin 84/10**

(84) Designated Contracting States:
**AT BE CH DE FR GB IT LU NL SE**

(56) References cited:
**DE - A - 2 328 175**
**DE - A - 2 537 620**
**FR - A - 2 263 738**
**NL - A - 6 709 780**
**NL - A - 7 801 015**
**US - A - 3 405 711**
**US - A - 3 805 767**

(73) Proprietor: **Verschoof, Karel Johan Hendrik**
**J.W. Racerstraat 95**
**NL-7514 AG Enschede (NL)**

(72) Inventor: **Verschoof, Karel Johan Hendrik**
**J.W. Racerstraat 95**
**NL-7514 DE Enschede (NL)**
Inventor: **Smit, Jan Willem**
**Schumannlaan 31**
**NL-7522 KD Enschede (NL)**

(74) Representative: **Schumann, Bernard Herman Johan et al,**
**OCTROOIBUREAU ARNOLD & SIEDSMA**
**Sweelinckplein 1**
**NL-2517 GK The Hague (NL)**

Courier Press, Leamington Spa, England.

Contraceptive device

The invention relates to a contraceptive device for use in the oviducts consisting of an oblong connection element connecting two enlargements having rounded shapes. A contraceptive device of this kind is disclosed in German published Patent Application 23 28 175. This prior art contraceptive device is dimensioned such that the enlargement 4 has a substantially larger maximum cross-section area than the enlargement 2, in a plane transverse to the longitudinal axis of the connection element 3. Due to this shape of the mentioned prior art contraceptive device the forces exerted by the muscles and the tissue of the oviduct in which the device is placed tend to shift the device in the direction of the enlargement having the largest cross-section area. A stable, balanced positioning cannot be attained.

A major object of the invention, therefore, is to provide an improved contraceptive device which can be reliably and stably positioned and fixed in the oviducts. In view thereof the invention provides a contraceptive device of the kind set forth which is characterized in that the maximum cross-section areas of said enlargements in a plane transverse to the longitudinal axis of said connection element are substantially equal.

It is noted that from Dutch Patent Application 6709780 an intrauterine contraceptive device is known in which a core consists of an oblong connection element connecting two enlargements, said enlargements having maximum cross-section areas in a plane transverse to the longitudinal axis of said connection element which are substantially equal. Due to the fact that the oblong connection element is fully surrounded by strips which are highly expandable by water absorption the forces exerted on the element in situ prevent a stable positioning of the device, contrary to the invention.

For obtaining a good closure of the oviducts while preventing an appreciable irritation the device according to the invention may be characterized in that the largest diameters of said enlargements are within the range of 2—6 mm, the length of said oblong connection element being within the range of 2—14 mm and the diameter of said connection element being in the range of 0.4—0.8 times the largest diameters of said enlargements.

In order to follow temporary dilatations of the oviducts the enlargements may at least partly be of rubber elastic synthetic resin.

If the dilatations of the oviducts are prolonged the dilatations can be compensated if at least part of the device according to the invention is provided with at least a circular layer of a material which can highly expand by water absorption. An example of such material is a hydrogel, particularly polyhydroxyethyl-methacrylate, known under the trade name Polyhema.

In order to add a sperm-killing feature to the contraceptive device at least said enlargements may have a core of a material containing copper.

The axial fixation of the device will in an important degree be the result of the forces exerted by the muscle tissue of the uterus on the facing surfaces of the enlargements. The closure predominantly takes place at the largest diameter of the enlargements, but also in a not-neglectable degree by the pressure exerted by the muscle tissue of the uterus on the oblong connection element.

It is hereby remarked that the tissue surrounding the device tends to adapt itself to the shape of the device, i.e. provided the device is stably positioned in the oviduct the local pressure tends to be more or less equal along the length of the device: the device "grows into the tissue" and the fixation becomes gradually less dependent upon the cross-section areas of the enlargements. When after some substantial time the device is removed, its shape can be recognized while inspecting by means of an endoscope the oviduct wall. From the above it will be clear that a firm and stable positioning of the device immediately after its introduction into the oviduct is a requirement; this requirement cannot be fulfilled by the device according to DE 23 28 175 as the forces exerted by the tissue on the enlargements can never reach a stable balance.

The closure can be improved by providing the connection element with at least one circular rib having a smoothly rounded shape and having a smaller diameter than said enlargements.

In a specific embodiment the device is hollow and open at one end only. In this embodiment measuring instruments can be accommodated in the cavity within the device and tools may be applied to grip in it, e.g. to bring the device in its place or to remove it therefrom, or to handle it while applying a layer of highly expandable material.

Further features and characteristics of the invention will be explained in connection with the drawing in which some preferred embodiments of the contraceptive device and its use are depicted.

Fig. 1 shows a partly axial sectioned contraceptive device.

Fig. 2 shows a transverse section along the line II—II in fig. 1.

Fig. 3 and 4 show axial sections of modified embodiments of the device shown in fig. 1.

Fig. 5 shows the use of the contraceptive device according to the invention.

Fig. 6 shows an axial section of a modified embodiment of the device shown in fig. 1.

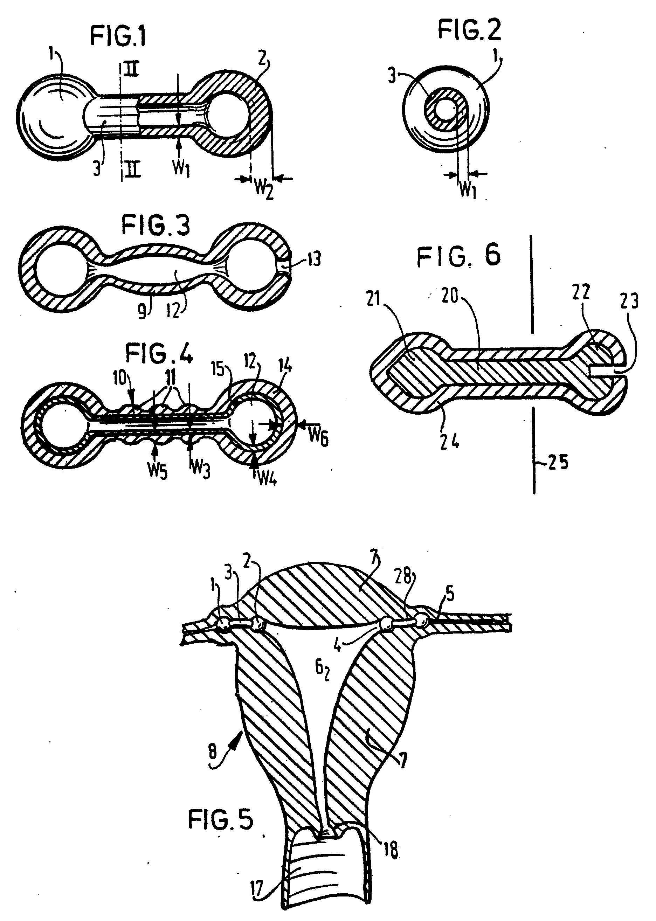

The contraceptive device as shown in fig. 1 comprises two spherical enlargements 1, 2 and a cylindrical oblong connection element 3. The device is entirely hollow. The wall thickness w 1 of the connection element and the wall thickness w 2 of the enlargements are dependent on the required elasticity of such parts.

In the depicted embodiment the ratio between the diameter d of the connection element 3 and the maximum diameter D of the enlargements 1, 2 is about 0,5, but this ratio can be varied in dependence on the circumstances from 0,4 to 0,8. A smaller ratio will prevent a seal on the connection element and a larger ratio will increase the risk of axial rejection from the mouth of the oviducts if an unequal muscle contraction occurs. The values of these diameters D and d and of the lengths l of the connection element will be dependent on the anatomic shape of the mouths 28 of the oviducts in the uterus and on the diameters of the oviducts just outside the uterus wall.

In fig. 5 the position of the contraceptive devices in the oviduct mouths 28 is shown: one of the enlargements still in the funnel shaped widened part 4 of the oviduct 5 in the uterus cavity and the other enlargement just outside the muscle tissue 7 of the uterus 8 in the oviduct. The several dimensions of the contraceptive device, particularly the length l of the connection element 3 have to be selected dependent on the anatomic data, in order that a suitable closure on all parts of the contraceptive device is obtained. The closure in the mouths 28 may be improved by providing the connection element with at least one circular rib as depicted in figs. 3 and 4. In fig. 3 the oblong element 9 is elliptic in longitudinal section, while in fig. 4 the oblong element 10 has three peripheral ribs 11 with rounded shapes for preventing tissue irritation.

Just like the embodiment as shown in fig. 1 that of fig. 3 is hollow, although in the latter case the internal cavity 12 debouches at one end through an opening 13 to the atmosphere enabling measuring instruments to be accommodated in the cavity and applying tools to grip in it.

The embodiment of fig. 4 is also different from that of fig. 1 in that a layered construction is used: the outer layer 14 may be a tissue friendly material, which property is not required for the material of the inner layer 15, which material may be chosen for its mechanical properties such as a certain elasticity.

The contraceptive device preferably is of at least an outer material which can highly increase in volume by water absorption, such as a hydrogel, offering the advantage that on dilatation of the cavity accommodating the device the diameter of the device will increase as a result of water absorption, so that the closure is safeguarded. After pressure increase the material can lose water and decrease diameter so that after a certain time the pressure exerted by the device on the surrounding tissues has an acceptable value again. Adapting the pressure is possible by a suitable selection of the materials of both layers and a selection of the wall thickness w 3 and w 4 of the inner layer 15 of the connection element and the enlargements respectively and the wall thickness w 5 and w 6 of the outer layer 14 of these parts.

In fig. 6 an axial section through a modified embodiment is shown, comprising an asymmetric core 20 made of copper and being rotary symmetric around its longitudinal axis. The core comprises a connection element and two enlargements at its two ends. The one enlargement 21 is slightly pointed at its free end for easier application, the other enlargement 22 being flattened at its free end and having an axial bore 23 for applying tools to grip in.

The core is coated all round with a layer of Polyhema (trade name) 24. Despite the asymmetric shape this embodiment satisfies the requirement that the maximum cross section areas of both enlargements in a plane 25 transverse to the longitudinal direction have to be equal.

It is evident that the contraceptive devices shown in fig. 3 and 6 are positioned with the opening 13, 23 respectively towards the uterus cavity 6.

**Claims**

1. A contraceptive device for use in the oviducts consisting of an oblong connection element (3) connecting two enlargements (1, 2) having rounded shapes, characterized in that the maximum cross-section areas of said enlargements (1, 2) in a plane transverse to the longitudinal axis of said connection element (3) are substantially equal.

2. The contraceptive device according to claim 1, characterized in that the largest diameters of said enlargements (1, 2) are within the range of 2—6 mm, the length of said oblong connection element (3) being within the range of 2—14 mm and the diameter of said connection element (3) being in the range of 0,4—0,8 times the largest diameter of said enlargements (1, 2).

3. The contraceptive device according to claims 1 or 2, characterized in at least part of it being provided with at least a circular layer of a material which can expand by water absorption.

4. The contraceptive device according to claim 3 characterized in that the entire device is made of expandable material.

5. The contraceptive device according to claims 3 or 4, characterized in that the expandable material is a hydrogel.

6. The contraceptive device according to any one of the preceding claims, characterized in that said enlargements (21, 22) have a core (20) of a material containing copper.

7. The contraceptive device according to any one of the preceding claims characterized in that the enlargements (1, 2) are at least partly of rubber elastic synthetic resin.

8. The contraceptive device according to any one of the preceding claims, characterized in that said oblong connection element (10) is provided with at least one circular rib having a smoothly rounded shape and having a smaller diameter than said enlargements (1, 2).

9. The contraceptive device according to any one of the preceding claims, characterized in that the device is hollow and open at one end only.

## Patentansprüche

1. Empfängnisverhütungsvorrichtung zur Benutzung in den Eileitern bestehend aus einem länglichen Verbindungselement (3), dass zwei Verdickungen (1, 2) mit einander verbindet, die abgerundete Formen aufweisen, dadurch gekennzeichnet, dass die maximalen Querschnittsflächen dieser Verdickungen (1, 2) in einer Ebene quer zur Längsachse des Verbindungselements im wesentlichen gleich sind.

2. Vorrichtung nach Anspruch 1, dadurch gekennzeichnet, dass der grösste Durchmesser der Verdickungen (1, 2) im Bereich von 2—6 mm, die Länge des länglichen Verbindungselements (3) im Bereich von 2—14 mm und der Durchmesser des Verbindungselements (3) im Bereich von 0,4—0,8 mal dem grössten Durchmesser der Verdickungen (1, 2) liegen.

3. Vorrichtung nach Anspruch 1 oder 2, dadurch gekennzeichnet, dass sie zumindest teilweise mit mindestens einer kreisförmigen Lage von Material umgeben ist, das sich durch Wasseraufnahme ausdehnt.

4. Vorrichtung nach Anspruch 3, dadurch gekennzeichnet, dass die Vorrichtung insgesamt aus sich ausdehnendem Material besteht.

5. Vorrichtung nach Anspruch 2 oder 4, dadurch gekennzeichnet, dass das sich ausdehnende Material ein Hydrogel ist.

6. Vorrichtung nach einem der vorhergehenden Ansprüche, dadurch gekennzeichnet, dass die Verdickungen (21, 22) einen Kern (20) aufweisen, der Kupfer enthält.

7. Vorrichtung nach einem der vorhergehenden Ansprüche, dadurch gekennzeichnet, dass die Verdickungen (1, 2) zumindest teilweise aus gummielastischem synthetischen Harz bestehen.

8. Vorrichtung nach einem der vorhergehenden Ansprüche, dadurch gekennzeichnet, dass das längliche Verbindungselement (10) mit mindestens einer ringförmigen Rippe (11) versehen ist, die eine sanft abgerundete Form und einen kleineren Aussendurchmesser als die Verdickungen (1, 2) aufweist.

9. Vorrichtung nach einem der vorhergehenden Ansprüche, dadurch gekennzeichnet, dass die Vorrichtung hohl ist und nur an einem Ende eine Öffnung aufweist.

## Revendications

1. Dispositif contraceptif, conçu pour être utilisé dans les trompes de Fallope, constitué par un élément de liaison oblong (3) reliant deux parties élargies (1, 2) ayant des formes arrondies, caractérisé en ce que les surfaces des sections droites maximales desdites parties élargies (1, 2), dans un plan transversal à l'axe longitudinal dudit élément de liaison (3) sont sensiblement égales.

2. Dispositif contraceptif selon la revendication 1, caractérisé en ce que les plus grands diamètres desdites parties élargies (1, 2) sont compris dans l'intervalle de 2 à 6 mm, la longueur dudit élément de liaison oblong (3) étant comprise dans l'intervalle de 2 à 14 mm et le diamètre dudit élément de liaison (3) étant compris dans l'intervalle de 0,4 à 0,8 fois les plus grandes parties desdites parties élargies (1, 2).

3. Dispositif contraceptif selon la revendication 1 ou 2, caractérisé en ce qu'au moins une partie dudit dispositif est munie d'au moins une couche circulaire d'une matière qui peut gonfler par absorption d'eau.

4. Dispositif contraceptif selon la revendication 3, caractérisé en ce que l'ensemble du dispositif est fabriqué en une matière gonflable.

5. Dispositif contraceptif selon la revendication 3 ou 4, caractérisé en ce que la matière gonflable est un hydrogel.

6. Dispositif contraceptif selon l'une quelconque des revendications précédentes, caractérisé en ce que lesdites parties élargies (21, 22) comprennent une âme (20) en une matière contenant dui cuivre.

7. Dispositif contraceptif selon l'une quelconque des revendications précédentes, caractérisé en ce que les parties élargies (1, 2) sont au moins en partie en une résine élastique synthétique caoutchouteuse.

8. Dispositif contraceptif selon l'une quelconque des revendications précédentes, caractérisé en ce que l'élément de liaison oblong (10) est muni d'au moins un renflement annulaire ayant une forme doucement arrondie et ayant un plus petit diamètre que lesdites parties élargies (1, 2).

9. Dispositif contraceptif selon l'une quelconque des revendications précédentes, caractérisé en ce que le dispositif est creux et ouvert seulement à une extrémité.

FIG.1

FIG.2

FIG.3

FIG. 6

FIG.4

FIG.5

1